# EUROPEAN PATENT APPLICATION

(11) **EP 1 304 373 A2**
(43) Date of publication of application: **23.04.2003**
(21) Application number: 02256937.0
(22) Date of filing: 07.10.2002
(51) Int. Cl.: C12N 9/64

(54) **Method for preparing a purified matrix metalloproteinase**

(30) Priority: 18.10.2001 US 348102 P
(71) Applicant: WARNER-LAMBERT COMPANY, Morris Plains, New Jersey 07950 (US)
(72) Inventor: Mueller, William Tomas, Pfizer Global R & D, Ann Arbor, Michigan 48105 (US); Pavlovsky, Alexander Grgory, Pfizer Global R & D, Ann Arbor, Michigan 48105 (US); Thanabal, Venkataraman, Pfizer Global R & D, Ann Arbor, Michigan 48105 (US); Yan, Chunhong, Pfizer Global R & D, Ann Arbor, Michigan 48105 (US)
(74) Representative: Motion, Keith Robert

(57) **Abstract**

This invention provides a method of preparing a purified MMP, the method comprising adding a ligand to a catalytic zinc cation of the MMP to a solution of a pro form of the MMP.

## Description

### BACKGROUND OF THE INVENTION

### (1) Field of the Invention

The present invention relates to a method for preparing a purified matrix metalloproteinase ("MMP") enzyme. A purified MMP is useful for screening for inhibitors of the MMP. MMP inhibitors are useful for the treatment of mammals that suffer from diseases responsive to inhibition of a matrix metalloproteinase. Such diseases include arthritis, multiple sclerosis, heart failure, stroke, and cancer, to name a few.

### (2) Description of the Related Art

The matrix metalloproteinases ("MMPs") are naturally occurring (i.e., endogenous) enzymes found in most, if not all, mammals. The catalytic activity of all MMPs is zinc-dependent. Examples of MMPs include collagenase-1 ("MMP-1"), also known as interstitial collagenase, gelatinase A ("MMP-2"), stromelysin-1 ("MMP-3"), matrilysin ("MMP-7"), collagenase-2 ("MMP-8"), also known as neutrophil collagenase, gelatinase B ("MMP-9"), metalloelastase ("MMP-12"), collagenase-3 ("MMP-13"), and membrane-type-1 MMP ("MT1-MMP" or "MMP-14"). Also included in the family of MMPs are truncated forms, including catalytic domains of MMPs.

One major biological function of the MMPs is to catalyze the breakdown of connective tissue or extracellular matrix by virtue of their abilities to hydrolyze various components of the tissue or matrix. Examples of the components that may be hydrolyzed by an MMP include collagens (e.g., type I, II, III, or IV), gelatins, proteoglycans, and fibronectins. For example, the collagenase sub-family of MMPs, namely MMP-1, MMP-8, and MMP-13, preferentially cleave collagens, and are thus usually associated with diseases linked to breakdown of collagen-based tissue. Another major biological function of the MMPs is proteolytic activation of the zymogen (pro-) forms of other MMPs, thereby inducing MMP activation.

Over-expression and/or over-activation of a matrix metalloproteinase ("MMP") or an imbalance between an MMP and a natural (i.e., endogenous) tissue inhibitor of a matrix metalloproteinase ("TIMP") has been linked to the pathogenesis of diseases characterized by the breakdown of connective tissue or extracellular matrix. Examples of diseases characterized by over-expression and/or over-activation of an MMP include rheumatoid arthritis, osteoarthritis, osteoporosis, periodontitis, multiple sclerosis, gingivitis, corneal, epidermal, and gastric ulceration, atherosclerosis, neointimal proliferation, which leads to restenosis and ischemic heart failure, stroke, renal disease, macular degeneration, and tumor metastasis.

Further, some MMP-mediated diseases may involve overactivity of only one MMP enzyme. This is supported by the recent discovery that MMP-13 alone is over-expressed in breast carcinoma, while MMP-1 alone is over-expressed in papillary carcinoma.

A purified MMP is useful for screening compounds to discover inhibitors of the MMP that are new and potent drug leads for treatment of human and animal diseases responsive to inhibition of the MMP.

Historically however, purification of an MMP, or a catalytic domain thereof, has been difficult. An MMP protein that has been produced, usually by expression in a cell line such as *Escherichia coli* ("*E. coli"*), has been purified by:
(1) collecting cells containing an expressed MMP protein by centrifugation;
(2) lysing the cells collected in step (1) to free the expressed MMP protein contained therein;
(3) cleaving extra amino acids from pro forms, if any, of the expressed MMP protein freed in step (2); and
(4) isolating MMP protein produced in step (3).
This purification process usually results in protein contaminated with impurities or protein that, because it is autolytic, degrades before it can be used for inhibitor screening or structure-activity relationship ("SAR") studies involving co-crystallization of an MMP bound with an inhibitor. Sometimes, this protein has insufficient specific activity for MMP inhibitor screening purposes.

For example, expression of MMP-13 catalytic domain ("MMP-13CD") in *E. coli* typically produces a pro form of the enzyme ("Pro-MMP-13CD") that has 3 extra amino acids, namely M-A-S-, on the N-terminal amino acid of MMP-13CD, which N-terminal amino acid is Y. This protein is named herein M-A-S-(MMP-13CD). The extra amino acids interfere with crystallization and purification of the enzyme. The M amino acid is typically removed by an *E. coli* enzyme to give a modified pro form of MMP-13CD, ("mPro-MMP-13CD"), which is named herein A-S-(MMP-13CD). The A and S amino acids are then removed by autolysis of the modified pro form A-S-(MMP-13CD), but unfortunately this autolysis does not stop at the mature MMP-13CD. The autolysis continues to trim amino acids beyond the mature MMP-13CD, rendering mixtures of truncated MMP-13CD protein forms that are not reproducible and have less than desirable characteristics such as, for example, purity or specific activity. A method of preparing purified MMP-13, or a catalytic domain thereof, would thus be particularly valuable.

The present invention provides a method of preparing a purified MMP enzyme. For example, purified MMP-13 catalytic domain and purified MMP-12 catalytic domain have been prepared in a pure, stable form using the method of the present invention. Surprisingly, addition of acetohydroxamic acid to the modified pro form A-S-MMP-13CD stimulates autolysis of the A and S amino acids, and then stabilizes the resulting mature MMP-13CD itself against autolysis. The method of the present invention works for any MMP enzyme or any form thereof, including full-length enzyme and truncated enzyme, including catalytic domains of an MMP. These and other advantages of this invention will be more fully described in the following text. All that is required to practice the invention method is to purify an MMP protein in the presence of a suitable amount of a ligand to the catalytic zinc cation of the protein.

### BRIEF SUMMARY OF THE INVENTION

This invention provides a method of preparing a purified matrix metalloproteinase enzyme. One embodiment of the present invention, hereinafter referred to as Method Embodiment 1, is a method of preparing a purified MMP, the method comprising adding a ligand to a catalytic zinc cation of the MMP to a solution of a pro form of the MMP.

Another embodiment of the present invention is the method according to Method Embodiment 1, wherein the MMP is MMP-13.

Another embodiment of the present invention is the method according to Method Embodiment 1, wherein the MMP is MMP-13 catalytic domain.

Another embodiment of the present invention is the method according to Method Embodiment 1, wherein the MMP is MMP-12.

Another embodiment of the present invention is the method according to Method Embodiment 1, wherein the MMP is MMP-12 catalytic domain.

Another embodiment of the present invention is the method according to Method Embodiment 1, wherein the ligand to a catalytic zinc cation of the MMP is a hydroxamic acid.

Another embodiment of the present invention is the method according to Method Embodiment 1, wherein the ligand to a catalytic zinc cation of the MMP is a hydroxamic acid which is acetohydroxamic acid.

Another embodiment of the present invention is the method according to Method Embodiment 1, wherein the MMP is MMP-13 catalytic domain and the ligand to the catalytic zinc cation is acetohydroxamic acid.

Another embodiment of the present invention is the method according to Method Embodiment 1, wherein the MMP is MMP-12 catalytic domain and the ligand to the catalytic zinc cation is acetohydroxamic acid.

Another embodiment of the present invention is the method according to Method Embodiment 1, further comprising adding a noncompetitive inhibitor of the MMP to the solution of the pro form of the MMP.

Another embodiment of the present invention is the method according to Method Embodiment 1, further comprising adding an uncompetitive inhibitor of the MMP to the solution of the pro form of the MMP.

Another embodiment of the present invention is the method according to Method Embodiment 1, further comprising the step of concentrating a solution of the purified MMP.

Another embodiment of the present invention is the method according to Method Embodiment 1, further comprising the step of isolating the purified MMP.

Another embodiment of the present invention is the method according to Method Embodiment 1, further comprising the step of isolating the purified MMP, wherein the isolation step comprises filtration of the solution of the purified MMP.

Another embodiment of the present invention is the method according to Method Embodiment 1, further comprising the step of isolating the purified MMP, wherein the isolation step comprises crystallizing the purified MMP.

Another embodiment of the present invention, hereinafter referred to as Method Embodiment 2, is a method of preparing a purified MMP, the method comprising:
(a) solubilizing pro form MMP inclusion bodies to give a solution of a pro form of the MMP; and
(b) adding a ligand to a catalytic zinc cation of the MMP to the solution of step (a).

Another embodiment of the present invention, hereinafter referred to as Method Embodiment 3, is a method of preparing a purified MMP, the method comprising:
(a) adding a ligand to a catalytic zinc cation of the MMP to a solution of a pro form of the MMP; and
(b) allowing the purified MMP produced in step (a) to crystallize.

Another embodiment of the present invention is the method according to Method Embodiment 1, 2, or 3, the method comprising:
(a) solubilizing pro form MMP inclusion bodies to give a solution of a pro form of the MMP;
(b) adding a ligand to a catalytic zinc cation of the MMP to the solution of step (a); and
(c) allowing the purified MMP produced in step (b) to crystallize.

Another embodiment of the present invention, hereinafter referred to as Method Embodiment 4, is a method of preparing a purified MMP, the method comprising:
(a) a step for preparing a purified MMP from a pro form of the MMP; and
(b) a step for isolating the purified MMP of step (a).

Another embodiment of the present invention is the method according to Method Embodiment 1, 2, 3, or 4, wherein the pro form of the MMP is an A-S-(MMP).

Another embodiment of the present invention, hereinafter referred to as Method Embodiment 5, is a method of inhibiting autolysis of a purified MMP, the method comprising adding a ligand to a catalytic zinc cation of the MMP to a solution of the purified MMP.

Another embodiment of the present invention is the method according to Method Embodiment 5, wherein the MMP is MMP-13.

Another embodiment of the present invention is the method according to Method Embodiment 5, wherein the MMP is MMP-13 catalytic domain.

Another embodiment of the present invention is the method according to Method Embodiment 5, wherein the MMP is MMP-12.

Another embodiment of the present invention is the method according to Method Embodiment 5, wherein the MMP is MMP-12 catalytic domain.

Another embodiment of the present invention is the method according to Method Embodiment 5, wherein the ligand to a catalytic zinc cation of the MMP is a hydroxamic acid.

Another embodiment of the present invention is the method according to Method Embodiment 5, wherein the ligand to a catalytic zinc cation of the MMP is a hydroxamic acid which is acetohydroxamic acid.

Another embodiment of the present invention is the method according to Method Embodiment 5, wherein the MMP is MMP-13 catalytic domain and the ligand to the catalytic zinc cation is acetohydroxamic acid.

Another embodiment of the present invention is the method according to Method Embodiment 5, wherein the MMP is MMP-12 catalytic domain and the ligand to the catalytic zinc cation is acetohydroxamic acid.

### DETAILED DESCRIPTION OF THE INVENTION

This invention provides a method of preparing a purified matrix metalloproteinase enzyme, the method comprising adding a ligand to a catalytic zinc cation of the MMP to a solution of a pro form of the MMP.

As used herein, the term "MMP" means matrix metalloproteinase, which includes a full-length MMP and a truncated MMP, including an MMP catalytic domain ("MMP CD"). An MMP contains a zinc (II) cation at its active site that is necessary for catalytic activity. The active site zinc cation acts by coordinating directly via a noncovalent bond, or indirectly via, for example, a bridging water molecule, or the conjugate acid or conjugate base thereof, to a functional group in a substrate that is being acted upon by the MMP.

It should be appreciated that the term "MMP", the phrase "MMP protein", and the phrase "MMP enzyme" are synonymous.

It should also be appreciated that the matrix metalloproteinases include, but are not limited to, the following full-length enzymes:
MMP-1, also known as interstitial collagenase, collagenase-1, or fibroblast-type collagenase;
MMP-2, also known as gelatinase A or 72 kDa Type IV collagenase;
MMP-3, also known as stromelysin or stromelysin-1;
MMP-7, also known as matrilysin or PUMP-1;
MMP-8, also known as neutrophil collagenase or polymorphonuclear-type ("PMN-type") collagenase;
MMP-9, also known as gelatinase B or 92 kDa Type IV collagenase;
MMP-10, also known as stromelysin-2;
MMP-11, also known as stromelysin-3;
MMP-12, also known as metalloelastase;
MMP-13, also known as collagenase-3;
MMP-14, also known as membrane-type ("MT") MMP-1 or MT-MMP-1;
MMP-15, also known as MT-MMP-2;
MMP-16, also known as MT-MMP-3;
MMP-17, also known as MT-MMP-4;
MMP-18; and
MMP-19.

The MMPs also include truncated forms of the full-length MMPs, including catalytic domains. Nonlimiting examples of catalytic domains of MMPs include:
MMP-1 catalytic domain ("MMP-1CD");
MMP-2 catalytic domain ("MMP-2CD");
MMP-3 catalytic domain ("MMP-3CD");
MMP-8 catalytic domain ("MMP-8CD");
MMP-9 catalytic domain ("MMP-9CD");
MMP-12 catalytic domain ("MMP-12CD"); and
MMP-13 catalytic domain ("MMP-13CD").

As used herein, "truncated forms" of an MMP mean those forms of the MMP that retain at least some of the catalytic activity of the MMP.

The phrase "catalytic domain" means the domain containing the catalytic zinc cation of an MMP, wherein the MMP enzyme contains two or more domains. For example, the collagenases, of which MMP-13 is a member, have been reported to contain a signal peptide domain, a propeptide domain, a catalytic domain, and a hemopexin-like domain (Ye Qi-Zhuang, Hupe D., Johnson L., *Current Medicinal Chemistry,* 1996;3:407-418). A catalytic domain further includes truncated forms thereof, provided a truncated form retains at least some of the catalytic activity of the catalytic domain.

The phrase "pro form of the MMP" means a pro form of a matrix metalloproteinase, which includes a pro form of a full-length MMP and a pro form of a truncated MMP, including a pro form of an MMP catalytic domain. Pro forms of an MMP comprise the MMP and independently from 1 to 50 extra amino acid residues bonded at either the N-terminal or C-terminal of the MMP, or independently from 1 to 25 extra amino acid residues at the N-terminal and independently from 1 to 25 extra amino acid residues at the C-terminal of the MMP. Pro forms of MMPs include naturally occurring zymogen forms and forms produced by expression of the MMP in cells, for example, *E. coli,* wherein the expression system employed produces the MMP containing a few extra amino acids. Illustrative examples of a pro form of an MMP include M-A-S-(MMP-13CD) and A-S-(MMP-13CD), which are described above, A-S-(MMP-13), A-S-(MMP-3), M-A-S-(MMP-7), and the like. Illustrative examples described above employ M, A, and S amino acids, but in principle any amino acid may comprise the extra amino acids of the pro form of the MMP.

The phrase "pro form MMP inclusion bodies" means an intracellular-derived mass that contains a pro form MMP.

The term "Pro-MMP-13CD" means the pro form of MMP-13 catalytic domain which is M-A-S-(MMP-13CD), wherein the extra amino acids of the tripeptide M-A-S are bonded to the N-terminal amino acid of MMP-13CD, which N-terminal amino acid is Y.

The term "mPro-MMP-13CD" means the pro form of MMP-13 catalytic domain which is A-S-(MMP-13CD), wherein the extra amino acids of the dipeptide A-S are bonded to the N-terminal of MMP-13CD, which N-terminal amino acid is Y.

The term "M" when referring to an amino acid means methionine.

The term "A" when referring to an amino acid means alanine.

The term "S" when referring to an amino acid means serine.

The term "Y" when referring to an amino acid means tyrosine.

The phrase "ligand to a catalytic zinc cation of the MMP" means any compound that binds to a catalytic zinc cation of the MMP being employed. In principle, a ligand of any potency versus the MMP, concentration, or size will work in the method of the instant invention provided that the ligand is at a concentration such that some unbound pro form MMP is present and some bound pro form MMP is present, as the invention method requires some catalytic activity of the pro form of the MMP being purified.

In any of the embodiments of the instant invention described above, preferred is a ligand that is a low molecular weight (i.e., a molecular weight of from 30 to 750 atomic units) molecule. More preferred is a ligand that has a molecular weight of from 40 to 500 atomic units. Still more preferred is a ligand that has a molecular weight of from 50 to 250 atomic units.

Illustrative examples of the ligand include acetohydroxamic acid, acetic acid, propanoic acid, N-hydroxy-propanamide, acetoacetic acid, malonic acid, ethanethiol, 1,3-propanedithiol, N-hydroxy-benzamide, imidazole, 2-mercaptoethanol, cyanide, thiocyanate, 2,4,6-trihydroxy pyrimidine, and the like. Low molecular weight, known inhibitors of a particular MMP such as, for example, a dipeptide inhibitor may be used as the ligand.

In any of the embodiments of the instant invention described above, preferred is a concentration of the ligand of from about 0.5 to about 3 *K*_{d}, wherein *K*_{d} is the disassociation constant for the ligand-enzyme complex. More preferred is a concentration of the ligand that is from about 1 *K*_{d} to about 2 *K*_{d}. A concentration of the ligand that is about 1 *K*_{d} means that about 50% of the target enzyme is bound to the ligand and about 50% of the target enzyme is free (i.e., unbound). In any of the embodiments of the instant invention described above, preferred is a concentration of the ligand that provides from about 20% bound target enzyme to about 90% bound target enzyme. More preferred is a concentration of the ligand that provides from about 35% bound target enzyme to about 90% bound target enzyme. Still more preferred is a concentration of the ligand that provides from about 40% bound target enzyme to about 90% bound target enzyme.

The phrase "solution of a pro form of the MMP" means a mixture that comprises the pro form of the MMP and a solvent, wherein at least some of the pro form of the MMP is dissolved in the solvent. Illustrative examples of a solution of a pro form of the MMP include a suspension of the pro form of the MMP in a solution of the pro form of the MMP wherein 1 % of the pro form of the MMP is dissolved and 99% of the pro form of the MMP is suspended and a solution wherein 100% of the pro form of the MMP is dissolved. Illustrative examples of the solvent include 6M guanidine hydrochloride, 50 mM Tris hydrochloride, buffered to pH 7.6, and polyethylene glycol ("PEG").

The phrase "isolating the purified MMP" means a process of obtaining an MMP purified by the invention method as a solid, wherein the solid may contain some residual solvent, including buffer.

The term "SDS-PAGE" means SDS-polyacrylamide gel electrophoresis.

The phrase "hydroxamic acid" means a compound of formula (A) 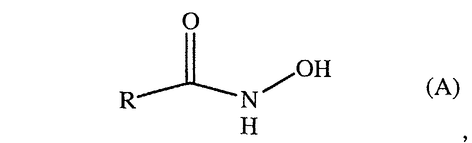 wherein
R is hydrogen, C₁-C₂₀ alkyl, C₂-C₂₀ alkenyl, C₂-C₂₀ alkynyl, phenyl, or benzyl.

The phrase "acetohydroxamic acid" means CH₃C(O)N(H)OH, which is synonymous with CH₃CONHOH, AcNHOH, and AcN(H)OH.

The term "alkyl" means: (i) a straight chain or branched chain hydrocarbon group having from 1 to 20 carbon atoms, (ii) a cyclic hydrocarbon group having from 3 to 20 carbon atoms, which is also known as a "cycloalkyl" group, (iii) a cyclic hydrocarbon group bonded through a straight chain or branched chain hydrocarbon group, which is also known as a "cycloalkyl-alkylene" group, wherein the total number of carbon atoms is from 4 to 20 and wherein alkylene is as defined below, or (iv) an alkyl group bonded through a cyclic alkylene, which is also known as an "alkyl-cycloalkylene" group, wherein the total number of carbon atoms is from 4 to 20 and wherein cycloalkylene is as defined below. Alkyl groups may be unsubstituted or substituted with from 1 to 3 fluoro. Preferred straight chain or branched chain alkyl groups have from 1 to 8 carbon atoms. Preferred cycloalkyl groups have from 3 to 8 carbon atoms. Other preferred alkyl groups have from 4 to 8 carbon atoms. C₁-C₆ alkyl means a straight chain or branched chain hydrocarbon group having from 1 to 6 carbon atoms. C₃-C₆ cycloalkyl means a cyclic hydrocarbon group having from 3 to 6 carbon atoms. Typical examples of straight chain or branched chain unsubstituted alkyl groups include methyl, ethyl, 1-propyl, 2-propyl, 1-butyl, 2-butyl, 2,2-dimethylethyl, 1-pentyl, 2-pentyl, 2,2-dimethylpropyl, 1-hexyl, 1-heptyl, 4-heptyl, 2-octyl, 2-methyl-hept-2-yl, 1-nonyl, 1-decyl, 1-undecyl, 1-dodecyl, 2-dodecyl, 2,4-dimethyl-2-decyl, 2-(1-methylethyl)-1-nonyl, 2-hexadecyl, and 1-tetradecyl. Illustrative examples of unsubstituted cycloalkyl groups are cyclopropyl, cyclobutyl, cyclopentyl, cyclohexyl, cycloheptyl, cyclooctyl, cyclononyl, cyclodecyl, cycloundecyl, cyclododecyl, cyclohexadecyl, and cyclotetradecyl. Illustrative examples of cycloalkyl-alkylene groups include cyclopropylmethyl, 3-cyclopentyl-hexyl, and 2-cyclopentyl-decyl. Illustrative examples of alkyl-cycloalkylene groups include 1-methyl-cyclopropyl, 3-hexyl-cyclopentyl, and 2-(dec-3-yl)-cyclopentyl. Substituted C₁-C₂₀ alkyl include CF₃, CF₃CH₂, and CH₃CF₂.

The term "alkenyl" means a straight chain or branched chain mono- or di-unsaturated hydrocarbon group having from 2 to 20 carbon atoms, or a cyclic mono-unsaturated hydrocarbon group having from 3 to 20 carbon atoms, which is also known as a "cycloalkenyl" group. Alkenyl groups may be unsubstituted or substituted with from 1 to 3 fluoro. Preferred straight chain or branched chain alkenyl groups have from 2 to 8 carbon atoms. Preferred cycloalkenyl groups have from 5 to 8 carbon atoms. Typical examples of straight chain or branched chain unsubstituted alkenyl groups include ethenyl, 1-propen-1-yl, 1-propen-2-yl, 2-propen-1-yl, 1-buten-3-yl, 1-butadienyl, 2-penten-2-yl, 1-hexen-6-yl, 1-hepten-3-yl, 3-hepten-1-yl, 2-octen-6-yl, 2-methyl-hept-2-en-4-yl, 1-nonen-8-yl, 1-decen-1-yl, 1-undecen-5-yl, CH₃-(CH₂)₁₀-C(H)=C(H)-C(H)=C(H)-, and 2,4-dimethyl-2-decen-1-yl. Illustrative examples of unsubstituted cycloalkenyl groups are 1-cyclopropenyl, 2-cyclobutenyl, 2-cyclopentenyl, 4-cyclohexenyl, 1-cycloheptenyl, 5-cyclooctenyl, 5-cyclononenyl, and 6-cyclotetradecenyl. Substituted C₂-C₂₀ alkenyl include CF₂=C(H) and CF₃-C(H)=C(H).

The term "alkynyl" means a straight chain or branched chain mono- or di-unsaturated hydrocarbon group having from 2 to 20 carbon atoms, or a cyclic mono-unsaturated hydrocarbon group having from 12 to 20 carbon atoms, which is also known as a "cycloalkynyl" group. Alkynyl groups may be unsubstituted or substituted with from 1 to 3 fluoro. Preferred straight chain or branched chain alkynyl groups have from 2 to 8 carbon atoms. Preferred cycloalkynyl groups have from 12 to 14 carbon atoms. Typical examples of straight chain or branched chain unsubstituted alkynyl groups include ethynyl, 1-propyn-1-yl, 1-propyn-3-yl, 2-propyn-1-yl, 1-butyn-3-yl, 1-butadiynyl, 2-pentyn-5-yl, 1-hexyn-6-yl, 1-heptyn-3-yl, 3-heptyn-1-yl, 2-octyn-6-yl, hept-2-yn-4-yl, and 4,4-dimethyl-2-decyn-1-yl. Illustrative examples of unsubstituted cycloalkynyl groups are 6-cyclotetradecynyl. Substituted C₂-C₂₀ alkynyl include F-C≡C and CF₃-C≡C.

It should be appreciated that di-unsaturated, straight chain or branched chain, alkenyl and alkynyl groups may have as the second site of unsaturation a C≡C or C=C bond, respectively.

The phrase "competitive inhibitor of the MMP enzyme" means an inhibitor of an MMP enzyme that competes with a ligand to a catalytic zinc cation of the MMP for access to the catalytic zinc cation. The binding of a competitive inhibitor or the ligand to the catalytic zinc cation of the MMP enzyme are mutually exclusive events. The potency of a competitive inhibitor to an MMP thus decreases as the concentration of substrate for the MMP increases.

The phrase "noncompetitive inhibitor of the MMP" means an inhibitor of an MMP enzyme that does not compete with the ligand to a catalytic zinc cation of the MMP for access to the catalytic zinc cation of the MMP enzyme or to the catalytic zinc cation of an MMP enzyme-substrate complex. A noncompetitive inhibitor does not prevent binding of a substrate to an MMP enzyme. A noncompetitive inhibitor acts by decreasing the turnover number of an enzyme (i.e., the number of substrate molecules converted to product by the enzyme, per unit time, when the enzyme is fully saturated with substrate).

A noncompetitive inhibitor acts by binding to an MMP enzyme, an MMP enzyme-substrate complex, or an MMP enzyme-product complex. The binding affinities of the noncompetitive inhibitor for the MMP enzyme and the MMP enzyme-substrate complex, or the MMP enzyme and the MMP enzyme-product complex, may be the same or different. A noncompetitive inhibitor that has different binding affinities for the MMP enzyme and the MMP enzyme-substrate complex, or the MMP enzyme and the MMP enzyme-product complex, is known as a "mixed" inhibitor of an MMP.

The phrase "uncompetitive inhibitor of the MMP" means an inhibitor of an MMP enzyme that does not compete with the ligand to a catalytic zinc cation of the MMP for access to the catalytic zinc cation of an MMP enzyme-substrate complex. An uncompetitive inhibitor of an MMP is an inhibitor that acts by binding to the MMP enzyme-substrate complex only.

The phrase "absolute intensity" or "a.i." means a unitless measure of relative ion intensity in a mass spectrum, and is determined by setting the tallest mass-to-charge ("m/z") peak in the spectrum as the base peak, and scaling every other m/z peak relative to the tallest peak.

Characterization of an inhibitor as noncompetitive or uncompetitive may be readily confirmed, if necessary or desired, by one skilled in the art by performing conventional steady-state kinetics experiments.

The term "about," when used herein to modify a number, means a numerical range that includes all values that may be rounded to the number being modified by the term. For example, in the above phrase "about 1 *K*_{d} to about 2 *K*_{d}", about 1 *K*_{d} means *K*_{d} in a range of from 0.5 to <1.5, which includes 0.5, 0.50001, and 1.4999999999 *K*_{d}. Each of 0.5, 0.50001, or 1.4999999999 may be rounded to the number being modified by about, which number is 1. Likewise, about 2 *K*_{d} means *K*_{d} in a range of from 1.5 to <2.5, and includes 1.5, 1.50001, and 2.4999999999 *K*_{d}. Accordingly, the phrase "about 1 *K*_{d} to about 2 *K*_{d}" includes *K*_{d} values in the range of from 0.5 to 2.49. Further examples of the use of the term about include "about 50%," which means a range of from 45% to <55%, and about pH 7.6, which means a range of from pH 7.55 to <pH 7.65.

The term "comprising" which is synonymous with the terms "including," "containing," or "characterized by" is inclusive or open-ended, and does not exclude additional, unrecited elements, or method steps from the scope of the invention that is described following the term.

The phrase "consisting of" is closed-ended, and excludes any element, step, or ingredient not specified in the description of the invention that follows the phrase.

The phrase "consisting essentially of" limits the scope of the invention that follows to the specified elements, steps, or ingredients, and those further elements, steps, or ingredients that do not materially affect the basic and novel characteristics of the invention.

The invention method for a particular MMP employs a suitable ligand to the active site zinc cation of the MMP. This ligand is thus a competitive inhibitor of the MMP. The ligand is identified by screening suitable compounds, preferably low molecular weight compounds, against the MMP in the presence of a suitable substrate, using a conventional assay method.

The ligand may be first buffered to the assay pH before it is added to the solution of the MMP.

The particular method described below uses the catalytic domain of the MMP-13 enzyme, namely matrix metalloproteinase-13 catalytic domain ("MMP-13CD"), rather than the corresponding full-length enzyme, MMP-13. It has been shown previously by Ye Qi-Zhuang, Hupe D., and Johnson L. *(Current Medicinal Chemistry,* 1996;3:407-418) that inhibitor activity against a catalytic domain of an MMP is predictive of the inhibitor activity against the respective full-length MMP enzyme.

The following examples are provided merely to further illustrate the invention. The scope of the invention is not to be construed as merely consisting of the following examples.

### PREPARATION 1

### Solubilization of mPro-MMP-13CD inclusion bodies

mPro-MMP-13 catalytic domain ("MMP-13CD") inclusion bodies were solubilized in 25 mL of 6 M guanidine hydrochloride and 50 mM Tris hydrochloride buffer at pH 7.7, by rotation of the mixture at room temperature overnight. The resulting solubilized mPro-MMP-13CD was centrifuged (SS-34 rotor, 15,000 ("15K") rpm) for 30 minutes, and the resulting supernatant was immediately placed in a freezer at -70°C.

### PREPARATION 2

### Diafiltration refolding of MMP-13CD

An aliquot (0.72 mL) of the -70°C supernatant from Preparation 1 was thawed and diluted in 400 mL of 6 M guanidine hydrochloride and 50 mM of Tris hydrochloride buffer at pH 7.6. The resulting solution was loaded into a 53Y10 diafiltration unit and cooled to 4°C by immersion in a chiller bath at a temperature of from about -15°C to about -10°C. The solution was diafiltered against 3.5 L of 50 mM Tris hydrochloride buffer at pH 7.6, 10 mM calcium chloride, 0.1 mM zinc chloride, and 10% glycol. After diafiltration and a rinse, the solution was filtered through a 0.2 µm filter to give 450 mL of solution containing mPro-MMP-13CD.

### PREPARATION 3

### Dialysis refolding of MMP-13CD

mPro-MMP-13CD may be refolded using a dialysis refolding procedure: A solution (1.22 mL) of solubilized mPro-MMP-13CD inclusion bodies, prepared as described above for Preparation 1, was diluted to 500 mL in 6 M guanidine hydrochloride, 50 mM Tris·HCl, pH 7.6 (estimated A₂₇₅ = 0.2). The protein was refolded by dialysis, 2 x 50 L 50 mM Tris·HCl, 10 mM CaCl₂, 0.1 mM ZnCl₂, overday and overnight. The solution was filtered through a 0.2 µm filter, and the filtrate was concentrated to 50 mL with a YM10 membrane at 4°C (A₂₇₇ = 1.21, 0.93 mg/mL). The solution of refolded mPro-MMP-13CD was flash-frozen at -80°C.

### PREPARATION 4

### Isolation of mPro-MMP13CD inclusion bodies

(1) Resuspend 650 g of *E. coli* cell paste containing Pro-MMP-13CD or mPro-MMP-13CD in 1.0 L of 50.0 mM Tris-HCl, pH 8.0;
(2) Add 10 mM MgCl₂ and 40.0 µL benzonase (EM Industries #1016569M) to the suspension of step (1), and stir at 4°C for 1.5 hours;
(3) Centrifuge the mixture of step (2) at 13,600 × G for 45.0 minutes at 4°C, and decant the supernatant (store about 0.5 mL of supernatant at -70°C for SDS-PAGE analysis);
(4) Resuspend the pellets produced in step (3) thoroughly with 1.0 L of 50.0 mM glycine-NaOH/10.0 mM EDTA pH 10.0 and 1.0% Triton X-100, and stir at 4°C overnight;
(5) Centrifuge the mixture of step (4) at 13,600 × G for 45.0 minutes at 4°C, and decant the supernatant (store about 0.5 mL of supernatant at -70°C for SDS-PAGE analysis);
(6) Resuspend the pellets produced in step (5) thoroughly with 1.0 L of 50.0 mM glycine-NaOH/10.0 mM EDTA pH 10.0 and 1.0% Triton X-100, and stir at 4°C overday;
(7) Centrifuge the mixture of step (6) at 13,600 × G for 45.0 minutes at 4°C, and decant the supernatant (store about 0.5 mL of supernatant at -70°C for SDS-PAGE analysis);
(8) Resuspend pellets produced in step (7) thoroughly with 1.0 L of 50.0 mM glycine-NaOH/10.0 mM EDTA pH 10.0 and 1.0% Triton X-100, and stir at 4°C overnight;
(9) Centrifuge the mixture of step (8) at 13,600 × G for 45.0 minutes at 4°C, and decant the supernatant (store about 0.5 mL of supernatant at -70°C for SDS-PAGE analysis);
(10) Resuspend pellets produced in step (9) thoroughly with 1.0 L 50.0 mM glycine-NaOH/10.0 mM EDTA pH 10.0, and stir at 4°C for 1.0 hours;
(11) Centrifuge the mixture of step (10) at 13,600 × G for 45.0 minutes at 4°C, and decant the supernatant (store about 0.5 mL of supernatant at -70°C for SDS-PAGE analysis);
(12) Resuspend pellets produced in step (11) thoroughly with 1.0 L of 50.0 mM Tris·HCl, pH 8.0 and 10.0 mM DTT, and stir at 4°C for 1.0 hours;
(13) Centrifuge the mixture of step (12) at 13,600 × G for 45.0 minutes at 4°C, and decant the supernatant (store about 0.5 mL of supernatant at -70°C for SDS-PAGE analysis);
(14) Resuspend pellets produced in step (13) thoroughly with 1.0 L Milli-Q water, and stir at room temperature for 30.0 minutes; and
(15) Centrifuge the mixture of step (12) at 13,600 × G for 45.0 minutes at 4°C, and decant the supernatant (store about 0.5 mL of supernatant at -70°C for SDS-PAGE analysis) to give mPro-MMP-13CD inclusion body pellets.

### PREPARATION 5

### Solubilization of mPro-MMP13CD inclusion bodies

This procedure is carried out at room temperature. Add 50 mL of solubilization buffer (50 mM Tris·HCl/6 M guanidine HCI, pH 7.6) to mPro-MMP-13CD inclusion body pellets obtained in Preparation 4, vortex at room temperature for 5 minutes, allow to sit for 5 more minutes, vortex again, repeat 3 times, to give a solution. Then, ultraviolet-visible ("UV-Vis") spectrometry is run to determine the amount of protein (extinction coefficient: 1.55). The solubilized material is chromatographed (sephacryl 100 (S 100) column, self-packed 700 mL Amicon column, roughly 32 mm × 87 cm, running speed about 2 mL/minute, loading is about 20 mL (2%-3% CV) per run) to remove any color and any aggregate, prior to the refolding process. There are several components that elute from the chromatography, the last component is mPro-MMP-13CD. However, all fractions should be run on SDS-PAGE prior to pooling. Fractions containing mPro-MMP-13CD are pooled, their concentration is estimated by UV-Vis spectrometry, and they are aliquoted into 2 to 3 mL fractions, which are stored at -80°C.

### PREPARATION 6

### CT18 refolding of mPro-MMP-13CD inclusion bodies

### Materials:

Guanidine HCl (USB, US75823, Lot 103791)
Trizma Base (Sigma, T-1503, Lot 90K5436)
Calcium Chloride (Sigma, C-3881, Lot 10K0196)
Zinc Chloride (Sigma, Z-3500, Lot 20K0263)
HCl (Mallinckrodt, 2062)

### Buffers:

Solubilization buffer: 50 mM Tri·HCl/6 M Guanidine HCl, pH 7.6 @ RT

Refolding buffer: 50 mM Tris·HCl, pH 7.6 at room temperature, add 10 mM CaCl₂ and 0.1 mM ZnCl₂ (add ZnCl₂ right before dialysis); 2 × 200 L, chill over the weekend at 4°C.

### Refolding procedure:

(1) The solubilized mPro-MMP-13CD CT18 is diluted with 2 L of solubilization buffer to about 0.1 mg/mL at room temperature, and protein concentration is determined by UV-Vis spectrometry at room temperature;
(2) Transfer the sample of step (1) to dialysis bags (Spectro/Por 3, 3500 MWCO, flat width 54 mm), and dialyze twice at 4°C: the first dialysis is at least 6 hours, and the second dialysis is for overnight; and
(3) After the 2 dialyses of step (2) are completed, concentrate the solution with YM10 membrane in a stirred cell to 0.8 to 1.0 mg/mL to give a concentrated solution of CT18-refolded mPro-MMP-13CD, and remove a 1 mL aliquot for analysis by 1D-NMR, MALDI mass spectrometry, and SDS-PAGE; the remaining solution is aliquoted into 2 mL fractions, flash-frozen, and stored at -80°C.

### EXAMPLE 1

### Purification of MMP-13CD

A 225 mL aliquot of the solution from Preparation 2 was combined with 3-benzyl-1-methyl-2,4-dioxo-1,2,3,4-tetrahydro-quinazoline-6-carboxylic acid benzyl ester (0.7 mg dissolved in 1 mL of dimethylsulfoxide ("DMSO") and acetohydroxamic acid (1.69 gm), and the resulting solution was stirred overnight at 4°C to give purified MMP-13CD. The remaining 225 mL of solution was combined with 4-benzyl-5-oxo-4,5-dihydro-[1,2,4]triazolo[4,3-a]quinazoline-7-carboxylic acid benzyl ester (0.8 mg dissolved in 1 mL of DMSO) and 1.69 g of acetohydroxamic acid, and the resulting solution was stirred overnight at 4°C to give purified MMP-13CD.

### EXAMPLE 2

### Purification of MMP-13CD

A 1 mL aliquot of the solution obtained in Preparation 3 was thawed and purified by addition of 100 µL of hydroxamic acid to give a solution of purified MMP-13CD, and the solution was stored at 4°C.

### EXAMPLE 3

### Mass spectrometry method

Matrix-Assisted Laser Desorption/Ionization Mass Spectrometry ("MALDI"): MALDI mass spectra were acquired on a Bruker Daltonics, Inc (Billerika, MA) Reflex-III time-of-flight mass spectrometer operating in linear mode. The instrument has a 3.3-meter flight tube and an adjustable accelerating voltage up to 23 kV in Delayed Extraction mode. Radiation from a Laser Science, Inc. (Newton, MA) nitrogen laser (337 nm, 3-nanosecond pulse width) was used to desorb ions from the target. All linear delayed-extraction experiments were performed using an operating voltage of 23.0 kV, an extraction grid voltage of 20.0 kV, a focus voltage of 16.8 kV, and a lens voltage of 9.001 kV. Twenty-five to one hundred-fifty laser shots were averaged for each spectrum with a 5-point Savitsky-Golay smooth applied. Samples were spotted (0.6 µL) onto a 384-spot sample target plate 1:1 [sample/3,5-dimethoxy-4-hydroxycinnamic acid 3:1 (0.1% trifluoroacetic acid/acetonitrile)], which was then allowed to air dry at room temperature.

Samples of protein were then analyzed by MALDI-MS every day for 5 days. The results were plotted graphically with the X-axis being mass-to-charge ("m/z", or mass per unit ionic charge) and the Y-axis being absolute intensity ("a.i."). Starting at Day 0, and continuing on Days 1 to 5, a 100 µL aliquot was removed, flash-frozen with liquid nitrogen, and stored at -80°C. The flash-frozen material was stored, then thawed and immediately analyzed. The mass-to-charge for MMP-13CD was about 18750 to about 18760. The major impurity in the solution of Preparation 3 had m/z of 18923. Other impurities, including mPro-(MMP-13CD), were observed in the solution of Preparation 3 between m/z 18500 and m/z 19500. M-A-S-(MMP-13CD) has a mass-to-charge in MALDI analysis of m/z = 19048; A-S-(MMP-13CD) has m/z = 18917; and MMP-13CD has m/z = 18759. Lower m/z impurities in the solution of Preparation 3 with a.i. values >400 were observed at m/z 9470, between 9400 and 9469, between about 9400 and 9365, 9365, 8431, 5868, 5021, 4869, 3733, between 3447 and about 3510, 3446, 2732, 2472, between 1900 and 2000, 1815, and 1671. After addition of acetohydroxamic acid, preparation of purified MMP-13CD was achieved. Results are shown below in Table 1 in the columns labeled "Impurity (m/z)" and "Ratio (a.i. of MMP-13CD/a.i. of impurity)" for Preparation 3 and Example 3 at Days 0 and 5. The data in the Ratio column is relative peak height for the MMP-13CD species at m/z 18923 versus the relative peak height for a particular impurity with an a.i. value that was greater than 500. The relative peak heights were calculated by dividing the a.i. value for MMP-13CD at m/z 18923 by the a.i. value for the particular impurity. The higher the ratio, the lower the amount of impurity relative to MMP-13CD.

| Ratio (a.i. of MMP-13CD/a.i. of impurity) | | | |
|---|---|---|---|
| Impurity (m/z) | Preparation 3^{a} | Example 3, Day 0^{b} | Example 3, Day 5^{c} |
| 18964 | N/M^{d} | N/O^{e} | 6.0 |
| 18923 | 1.2 | N/O | N/O |
| 18915 | N/M | 3.5 | N/O |
| 18588 | N/M | N/O | 6.2 |
| 9470 | 3.4 | N/O | N/O |
| Between 9400 and 9469 | 3.5 | N/O | N/O |
| Between about 9400 and 9365 | 4.8 | 3.0 | 2.8 |
| 9365 | 3.5 | N/O | N/O |
| 8431 | 7.1 | N/O | N/O |
| 5868 | 2.4 | N/O | N/O |
| 5021 | 3.6 | 2.6 | N/O |
| 4869 | 4.5 | N/O | N/O |
| 4003 | N/O | 3.9 | N/O |
| 3733 | 2.3 | N/O | N/O |
| 3593 | N/O | 2.6 | N/O |
| Between 3447 and about 3510 | 3.5 | N/O | N/O |
| 3446 | 2.1 | N/O | N/O |
| 2732 | 2.0 | N/O | N/O |
| 2472 | 4.2 | N/O | N/O |
| Between 1900 and 2000 | 5.6 | N/O | N/O |
| 1815 | 4.5 | N/O | N/O |
| 1671 | 2.0 | N/O | N/O |

| | | | |
|---|---|---|---|
| ^{a} MMP-13CD m/z 18755 peak height = 2900 a.i. | | | |
| ^{b} MMP-13CD m/z 18758 peak height = 2080 a.i., data taken after addition of acetohydroxamic acid. | | | |
| ^{c} MMP-13CD m/z 18752 peak height = 3750 a.i. | | | |
| ^{d} N/M means not measurable. | | | |
| ^{e} N/O means peak above 500 a.i. was not observed. | | | |

As seen in Table 1 above, the number of impurities with peak heights >500 a.i. in the solution of MMP-13CD of Preparation 3 decreased from an estimated 16 peaks before addition of acetohydroxamic acid to 1 peak by Day 5. Further, the impurities between about 18500 and about 19500 described above for Preparation 3 improved substantially by Day 5, as on Day 5 only 2 impurity peak heights above 500 a.i. near MMP-13CD were observed, and, as seen in Table 1, these impurity peaks were small relative to the peak height for MMP-13CD.

### EXAMPLE 4

### Purification of Refolded MMP-13

Thaw the flash-frozen CT18-refolded mPro-MMP-13CD protein (1 mg/mL) of Preparation 6, and transfer to a stirred cell with YM10 membrane (Amicon), add acetohydroxamic acid to give a final concentration of 100 mM of acetohydroxamic acid, and stir overnight at 4°C to give purified MMP-13CD.

### EXAMPLE 5

### Optional Further Purification of Purified MMP-13CD by Size Exclusion Chromatography

In the event that further purification of the purified MMP-13CD of Examples 3 or 4 is desired:
(1) Concentrate the purified MMP-13CD of Examples 3 or 4 in the stirred cell 10-fold by volume, to a final volume of 10 mL or less;
(2) Spin the concentrated sample of step (1) at 14000 rpm for 30 minutes at 4°C (SA 600 rotor), transfer the supernatant to a 50 mL centrifuge tube, and place the tube on ice, ready for injection onto column;
(3) Equilibrate a HiLoad 26/60 superdex 200 (Pharmacia Biotech Products) column in a Waters HPLC system by running 300 mL of 0.1N NaOH through at an elution rate of 1 mL/minute, followed by 500 mL of gel filtration buffer (50 mM Tris·HCl, pH 7.6, and 10 mM CaCl₂ and 0.1 mM ZnCl₂);
(4) Inject the supernatant of step (2) (should be less than 10 mL) through a 10 mL sample injection loop onto the equilibrated column of step (3), and elute with gel filtration buffer at a rate of 1 mL/minute for 350 minutes, collecting fractions every minute and monitoring the effluent by ultraviolet detector at 2 wavelengths (260 nm and 278 nm, typically a major peak comes off starting at about 260 minutes, peaking around about 270 to about 280 minutes, there is typically another peak that comes off right after the main peak, this peak having most absorption at 260 nm), to give further purified MMP-13CD; and
(5) Optionally pool the fractions containing the purified MMP-13CD of step (4), optionally estimate protein concentration (the extinction coefficient for MMP-13CD is 1.55) with a UV-Vis spectrophotometer, optionally aliquot the pooled fractions into measured volumes such as, for example, 2 mL aliquots as desired, and optionally flash-freeze the aliquots in liquid nitrogen and store at -80°C.

### EXAMPLE 6

### Refolding of the human MMP-12 catalytic domain ("hMMP-12CD") by dialysis and concentration

Material:
   Dialysis membrane (tubing) 10 kDa from Spectrum (ref Spectra/Por 7)
   Centriprep 10 kDa from Amicon (ref YM 10)
   Dialysis Buffer (+4°C): 25 mM Tris/HCl pH 7.5, 5 mM CaCl₂, 50 µM ZnCl₂, 100 mM NaCl, and 100 mM AcNHOH.
Procedure:
   (a) The pro form MMP sample of hMMP-12CD was diluted with 50 mM Gly/NaOH, pH 10, and 5 M urea buffer until the protein concentration reached about 0.1 mg/mL;
   (b) The 10 kDa dialysis membranes were filled in with the pro form MMP sample, such that the ratio sample volume/dialysis buffer volume was about 20;
   (c) Stirred the mixture of step (b) and dialyzed overnight at +4°C;
   (d) Changed the dialysis buffer of step (c), and dialyzed for 4 hours at 4°C with stirring;
   (e) Changed the dialysis buffer of step (d), and dialyzed for 4 hours at 4°C with stirring;
   (f) Concentrated the dialysate of step (e) in Centripep 10 kDa, making sure the concentration of purified hMMP-12CD remained at less than or equal to 2 mg/mL;
   (g) Analyzed purity of the hMMP-12CD produced in step (f) by SDS-PAGE using gel stained by SyproOrange and revealed on the storm, which showed hMMP-12CD of step (f) was greater than or equal to 90% pure; and
   (h) Stored the purified human MMP-12CD of step (f) at -20°C.

Autodegradation of the purified hMMP-12CD was observed if the protein was stored at +4°C for several days (about 50% degradation after 6 days).

The purified MMP-13CD obtained in Examples 3 or 4, or Example 5, step (5) or step (6), and the purified MMP-12CD obtained in Example 6, are useful for inhibitor screening, enzyme crystallization, or enzyme-inhibitor co-crystallization studies, for example.

An MMP enzyme purified according to the method of the present invention is useful for the following studies: (i) preparation of inhibitor-bound MMP crystals for x-ray crystallography studies, (ii) preparation of active: of an MMP weeks and months ahead of research studies rather than having to prepare MMP material immediately prior to use, and (iii) use in MMP inhibitor screening efforts, which screening efforts are enhanced over standard methods because the MMP enzyme produced by the invention method typically possesses higher specific activity than MMP produced by standard methods, and thus the screening data is more informative to a skilled medicinal chemist and more reliable. The purified MMP enzymes produced according to the invention method are suitable for high throughput screening.

A purified MMP stabilized according to a method of the present invention is stable in solution for prolonged periods, and is thus useful in the above studies.

The method of the instant invention works for preparing a purified MMP of any form, including a full-length MMP or a truncated form thereof, including a catalytic domain, or a truncated form thereof. All that is required for the invention method is autolytic activity of a pro form of the MMP being prepared in purified form.

Having described the present invention above, embodiments of the invention method are hereupon claimed.

## Claims

1. A method of preparing a purified MMP, the method comprising adding a ligand to a catalytic zinc cation of the MMP to a solution of a pro form of the MMP.

2. The method according to Claim 1, further comprising the step of adding a noncompetitive inhibitor of the MMP to the solution of the pro form of the MMP; or
The method according to Claim 1, further comprising the step of adding an uncompetitive inhibitor of the MMP to the solution of the pro form of the MMP; or
The method according to Claim 1, further comprising the step of concentrating the solution of the purified MMP; or
The method according to Claim 1, further comprising the step of isolating the purified MMP; or
The method according to Claim 1, further comprising the step of isolating the purified MMP, wherein the isolation step comprises filtration of the solution of the purified MMP; or
The method according to Claim 1, further comprising the step of crystallizing the purified MMP.

3. A method of preparing a purified MMP, the method comprising:
(a) solubilizing pro form MMP inclusion bodies to give a solution of a pro form of the MMP; and
(b) adding a ligand to a catalytic zinc cation of the MMP to the solution of step (a).

4. A method of preparing a purified MMP, the method comprising:
(a) adding a ligand to a catalytic zinc cation of the MMP to a solution of a pro form of the MMP; and
(b) allowing the purified MMP of step (a) to crystallize.

5. A method of preparing a purified MMP, the method comprising:
(a) solubilizing pro form MMP inclusion bodies to give a solution of a pro form of the MMP;
(b) adding a ligand to a catalytic zinc cation of the MMP to the solution of step (a); and
(c) allowing the purified MMP of step (b) to crystallize.

6. The method according to any one of Claims 1, 3, 4, and 5, wherein the MMP is MMP-13; or
The method according to any one of Claims 1, 3, 4, and 5, wherein the MMP is MMP-13 catalytic domain; or
The method according to any one of Claims 1, 3, 4, and 5, wherein the MMP is MMP-12; or
The method according to any one of Claims 1, 3, 4, and 5, wherein the MMP is MMP-12 catalytic domain; or
The method according to any one of Claims 1, 3, 4, and 5, wherein the ligand to a catalytic zinc cation of the MMP is a hydroxamic acid; or
The method according to any one of Claims 1, 3, 4, and 5, wherein the ligand to a catalytic zinc cation of the MMP is a hydroxamic acid which is acetohydroxamic acid; or
The method according to any one of Claims 1, 3, 4, and 5, wherein the MMP is MMP-13 catalytic domain and the ligand to the catalytic zinc cation is acetohydroxamic acid; or
The method according to any one of Claims 1, 3, 4, and 5, wherein the MMP is MMP-12 catalytic domain and the ligand to the catalytic zinc cation is acetohydroxamic acid.

7. The method according to any one of Claims 1 to 6, wherein the pro form of the MMP is an A-S-(MMP).

8. A method of inhibiting autolysis of a purified MMP, the method comprising adding a ligand to a catalytic zinc cation of the MMP to a solution of the purified MMP.

9. The method according to Claim 8, wherein the MMP is MMP-13; or
The method according to Claim 8, wherein the MMP is MMP-13 catalytic domain; or
The method according to Claim 8, wherein the MMP is MMP-12; or
The method according to Claim 8, wherein the MMP is MMP-12 catalytic domain; or
The method according to Claim 8, wherein the ligand to a catalytic zinc cation of the MMP is a hydroxamic acid; or
The method according to Claim 8, wherein the ligand to a catalytic zinc cation of the MMP is a hydroxamic acid which is acetohydroxamic acid; or
The method according to Claim 8, wherein the MMP is MMP-13 catalytic domain and the ligand to the catalytic zinc cation is acetohydroxamic acid; or
The method according to Claim 8, wherein the MMP is MMP-12 catalytic domain and the ligand to the catalytic zinc cation is acetohydroxamic acid.
